# EUROPEAN PATENT APPLICATION

(11) **EP 4 606 314 A1**
(43) Date of publication of application: **27.08.2025**
(21) Application number: 23880166.6
(22) Date of filing: 17.10.2023
(51) Int. Cl.: A61B 8/00, A61B 8/12, A61B 5/00, A61B 8/08, G01S 15/89

(54) **ELECTRICAL NETWORK FOR PHOTOACOUSTIC-ULTRASONIC PROBE, AND PHOTOACOUSTIC-ULTRASONIC PROBE HAVING SAME**

(30) Priority: 17.10.2022 KR 20220133573; 13.10.2023 KR 20230136979
(71) Applicant: Ulsan National Institute of Science and Technology (UNIST), Eonyang-eup Ulju-gun Ulsan 44919 (KR)
(72) Inventor: YANG, Joon-Mo, Ulju-gun, Ulsan 44919 (KR)
(74) Representative: Studio Torta S.p.A.
(86) International application number: PCT/KR2023/016015
(87) International publication number: WO 2024/085589

(57) **Abstract**

The present disclosure proposes: a new network capable of solving the problem of voltage signal level imbalance between photoacoustic and ultrasonic signals when collecting data in conventional single element transducer-based, mechanical scan method-type optical-resolution integrated photoacoustic-ultrasonic endoscopes or mini-probes or catheter devices; and a concept enabling the effective triggering of a data collection device, i.e., a digitizer, without loss of relevant data.

## Description

### Technical Field

The present disclosure relates to a medical tomographic endoscopic device that is implemented in the form of a thin and long probe like an ultrasound endoscope currently used in clinical practice and is inserted into a subject (or patient) to capture tomographic images of a surrounding region.

### Background Art

The present disclosure relates to a so-called integrated photoacoustic and ultrasonic endoscopy (PAE-EUS) technology that may simultaneously provide photoacoustic imaging information while maintaining a function of the traditional endoscopic ultrasound.

### Disclosure of Invention

### Technical Problem

Previously, it was not possible to solve the problem of signal imbalance that may occur when simultaneously collecting a photoacoustic signal and an ultrasonic signal by using a single digitizer.

### Solution to Problem

In the present disclosure, a unique circuit is configured to amplify photoacoustic signals and ultrasonic signals with independent gain values based on an ultrasonic T/R switch, an RF switch, and variable amplifiers, and a method is devised to match the dynamic ranges of both signals to similar levels after amplification processing. In addition, in the process of obtaining A-line signals for a photoacoustic signal and an ultrasonic signal at each step of a scanning tip, a method of inputting only one trigger pulse to the digitizer instead of the general two pulse inputs is applied, and accordingly, a frequency (trigger rate) for triggering the digitizer may be significantly reduced to half of the existing one.

### Advantageous Effects of Invention

By applying an independent amplification circuit of a photoacoustic signal and ultrasonic signal proposed in the present disclosure, a desired photoacoustic-ultrasonic dual-mode imaging sequence may be performed more quickly and stably without data loss problem caused by either one of the amplified photoacoustic and ultrasonic A-line signals exceeding a dynamic range of a digitizer or by a digitizer trigger frequency being applied too high.

### Brief Description of Drawings

FIG. 1 is a schematic diagram illustrating the entire concept of a signal amplification circuit that may be applied to a photoacoustic-ultrasonic probe device according to an embodiment of the present disclosure.
FIG. 2 is an embodiment of a related circuit implemented according to the concept presented in FIG. 1.
FIG. 3A and FIG. 3B are digitizer trigger sequences that may be applied to operate the embodiment presented in FIG. 2.

### Best Mode for Carrying out the Invention

The present disclosure may be modified in various ways and has various embodiments, and certain embodiments are illustrated in the drawings and described in detail. Effects and features of the present disclosure and a method for achieving the effects and features will become clear with reference to the embodiments described in detail below together with the drawings. However, the present disclosure is not limited to the embodiments disclosed below but may be implemented in various forms.

Hereinafter, embodiments of the present disclosure are described in detail with reference to the attached drawings, and when description is made with reference to the drawings, the same or corresponding components are assigned the same reference numerals and redundant descriptions thereof are omitted.

In the following embodiments, the terms first, second, and so on are not used in a limited sense but are used for the purpose of distinguishing one component from another component.

In the following embodiments, the singular expression includes the plural expression unless the context clearly indicates otherwise.

In the following embodiments, the terms "include" or "have" mean that a feature or component described in the specification exists, and do not preclude the possibility that one or more other features or components may be added.

In the embodiments below, when a component is said to be "connected" to another component, this includes not only being directly connected to the other component, but also being indirectly connected to the other component through another component.

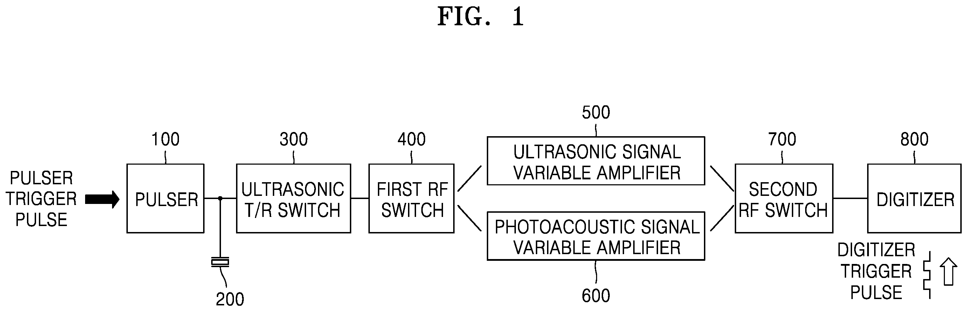

FIG. 1 is a schematic diagram illustrating the concept of a signal amplification circuit that is derived according to the present disclosure and applicable to various photoacoustic-ultrasonic probe devices.

Referring to FIG. 1, an ultrasonic transducer excitation and signal amplification circuit according to the present disclosure includes a pulser 100, an ultrasonic T/R switch 300, a first RF switch 400, an ultrasonic signal variable amplifier 500, a photoacoustic signal variable amplifier 600, and a second RF switch 700. The components described above are connected to a load referred to as an ultrasonic transducer 200, and accordingly, the ultrasonic transducer 200 may be provided between the pulser 100 and the ultrasonic T/R switch 300 as illustrated in FIG. 1. In addition, a digitizer 800 for collecting data may be connected to a final stage of a circuit. That is, among the components described above, an electric circuit including components other than the pulser 100 and the ultrasonic transducer 200 may be an electric circuit for the photoacoustic-ultrasonic probe of the present disclosure. Furthermore, a configuration including an electric circuit for the photoacoustic-ultrasonic probe, the pulser 100, and the ultrasonic transducer 200 may be referred to as the photoacoustic-ultrasonic probe of the present disclosure.

Referring to FIG. 1 to continuously describe the related operating concept, a step pulse generated at each step of a scanning tip (not illustrated) of the photoacoustic-ultrasonic probe is input to the pulser 100 illustrated in FIG. 1 in the form of a "pulser trigger pulse", and accordingly, the pulser 100 outputs a short and high-voltage electric pulse to the ultrasonic transducer 200 connected to an output terminal of the pulser 100. Then, the ultrasonic transducer 200, which can emit an ultrasonic wave and detect a reflected wave according thereto, transmits an electrical signal to the ultrasonic T/R switch 300, which is the next stage.

Also, after a pulse laser (not illustrated) emits a laser pulse to a subject without receiving the "pulser trigger pulse" described above, a photoacoustic signal detected by the ultrasonic transducer 200 is transmitted to the ultrasonic T/R switch 300. However, because the electric pulse and the laser pulse are emitted at different times, the ultrasonic T/R switch 300 appropriately switches an electrical signal reception mode to a high impedance mode and a low impedance mode according thereto. That is, the ultrasonic T/R switch 300 may be in the high impedance mode when receiving an electrical signal related to the ultrasonic wave and may be in the low impedance mode when receiving a photoacoustic signal related to the laser pulse. When alternately receiving an ultrasonic electrical signal and a laser pulse-related photoacoustic signal, the ultrasonic T/R switch 300 may repeatedly operate in the high impedance mode and the low impedance mode.

In addition, the first RF switch 400 appropriately transmits the ultrasonic signal and photoacoustic signal received from the ultrasonic T/R switch 300 respectively to the ultrasonic signal variable amplifier 500 and the photoacoustic signal variable amplifier 600 by referring to an emission point in time of the electric pulse and the laser pulse That is, the first RF switch 400 transmits the ultrasonic signal received from the ultrasonic T/R switch 300 to the ultrasonic signal variable amplifier 500, and transmits the photoacoustic signal received from the ultrasonic T/R switch 300 to the photoacoustic signal variable amplifier 600. Each variable amplifier that receives the signals amplifies each signal according to a preset amplification gain value. Also, the amplification gain value set for each variable amplifier is set such that voltage levels of the amplified photoacoustic signal and ultrasonic signal are similar to each other. That is, the voltage level of the ultrasonic signal amplified by the ultrasonic signal variable amplifier 500 may correspond to the voltage level of the photoacoustic signal amplified by the photoacoustic signal variable amplifier 600. The electrical signals amplified to a preset level by each variable amplifier are transmitted to the second RF switch 700, and then commonly collected by the digitizer 800 connected thereto.

For reference, the ultrasonic T/R switch 300, the first RF switch 400, or the second RF switch 700 used for the disclosure concept presented by the present specification are only example names, and may be replaced with components of different forms or names that perform the same functions as the functions described above. In addition, when the ultrasonic signal and the photoacoustic signal have a wide dynamic range depending on types and distances of a sound source, a non-linear amplifier, such as a log amplifier, may be used instead of a linear amplifier.

In addition, FIG. 1 only illustrates minimum components required to implement the core concept derived by the present disclosure, and a low-noise preamplifier (not illustrated) with a high-voltage pulse defense capability may be added between the ultrasonic transducer 200 and the ultrasonic T/R switch 300. In another embodiment, a low-noise preamplifier may be added between the ultrasonic T/R switch 300 and the first RF switch 400 to minimize the introduction of noise, and accordingly, a weak photoacoustic signal and an ultrasonic initial signal may be amplified at an earlier stage. For example, a low-noise preamplifier capable of defending against high-voltage pulses may include MAX4805 or so on made by the Maxim Integrated company, and because the ultrasonic T/R switches 300, the first RF switch 400, the second RF switch 700, or so on is widely used in the related field, descriptions of a detailed model are omitted. Also, a combination of the components according to the present disclosure is not previously known, and there is no suggestion of this. Furthermore, filters, capacitors, or so on may be added between signal processing stages formed by respective components illustrated in FIG. 1.

FIG. 1 is only an example, and when A-line data lengths of the photoacoustic and ultrasonic signals to be collected are significantly different from each other and the numbers of A-line data sets to be collected for each rotation of a scanning tip are different from each other, two or more digitizers may be independently connected to collect each signal differently instead of using one digitizer as illustrated in FIG. 1. In this case, the photoacoustic signal and the ultrasonic signal are individually collected and recorded by two independent digitizers, the number of A-line data sets to be collected for each rotation of the scanning tip may be easily set differently, and the number of pulses per time that trigger each digitizer may also be appropriately set differently for that purpose. Also, in this case, there is no need to add the second RF switch 700 that combines the photoacoustic signal and ultrasonic signal into one path (port) and transmits the signals to a subsequent stage.

### Mode for the Invention

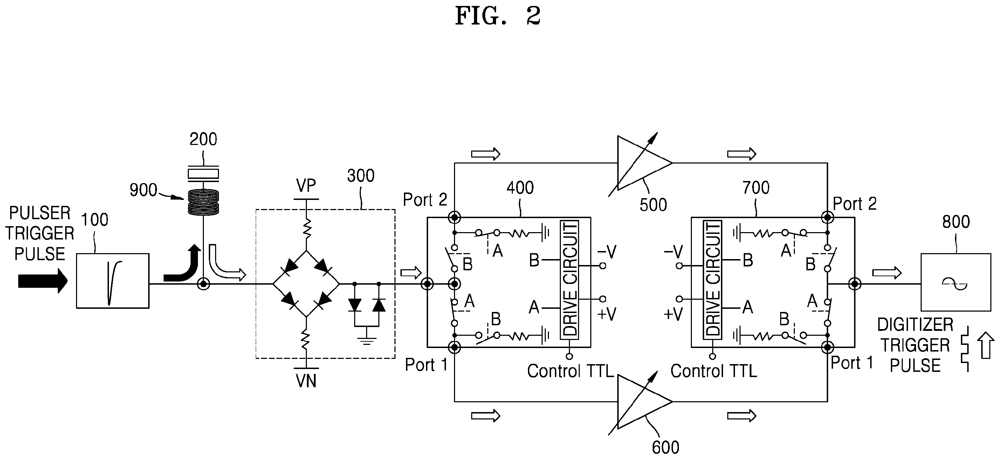

FIG. 2 is an embodiment of a related circuit that is a more specific implementation according to the concept presented in FIG. 1. An operating principle of the circuit is the same as the operating principle presented above with reference to FIG. 1 and is accordingly omitted, except that a rotary transformer 900 is additionally provided immediately before the ultrasonic transducer 200. In addition, the embodiment uses the first RF switch 400 and the second RF switch 700 each including an inner driver, that is, a drive circuit. For reference, hollow arrows drawn along wires indicate paths through which signals detected by the ultrasonic transducer 200 are transmitted, and symbols, such as +V, -V, VP, and VN indicate a type of drive voltages that have to be supplied to the related components.

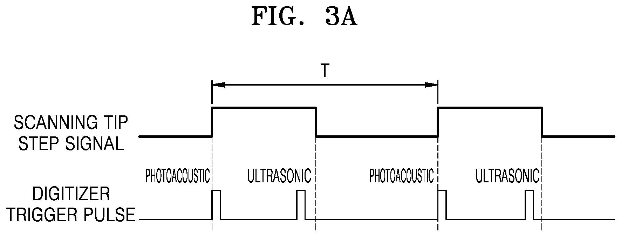

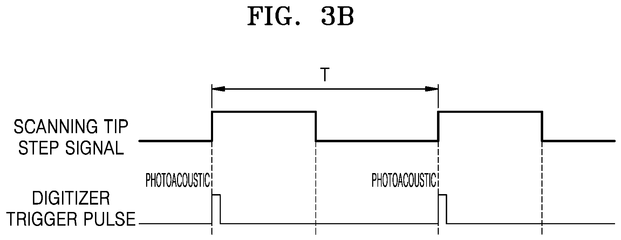

FIG. 3A and FIG. 3B illustrate trigger sequences of a digitizer that may be applied to operate the embodiment presented in FIG. 2. Based on this, the present disclosure proposes novel digitizer trigger sequences.

First, FIG. 3A illustrates a trigger method similar to a typical digitizer trigger method that has been applied to a conventional single transducer-based, mechanical scanning, integrated optical-resolution photoacoustic and ultrasonic endoscope, a probe, a mini probe, catheter or so on, to which the circuits of FIG. 1 and FIG. 2 proposed by the present disclosure also may be applied. As described above, one photoacoustic trigger signal and one ultrasonic trigger signal are generated for each step signal of each scanning tip to trigger the digitizer 800. As a result, the digitizer 800 is triggered at a frequency approximately twice the step frequency of the scanning tip. T illustrated in FIG. 3A is a parameter indicating a cycle.

FIG. 3B is a diagram illustrating a sequence for triggering the digitizer 800 by generating only one trigger pulse for each step signal of each scanning tip according to the present disclosure. That is, because one trigger pulse is used for each step signal, it is acceptable to input the same waveform as the step signal of the scanning tip to the digitizer 800 because in this case, it is still normally workable even without applying a trigger waveform having a low duty (<< 50 %) as illustrated in the FIG. 3B.

However, one thing to note here is that a trigger point of the digitizer 800 has to coincide with or be synchronized with an emission point in time of the laser pulse emitted for each step, and as there is an independent digitizer trigger point in time for recording an ultrasonic signal in FIG. 3A, a process of emitting an ultrasonic pulse at a preset point in time ahead of the trigger point in time in a shape synchronized therewith has to be also included naturally. In other words, only the signal indicating that the ultrasonic signal is recorded to the digitizer is not given, but the process of emitting the laser pulse and the ultrasonic pulse have to be maintained as in the case of FIG. 3A.

The reason why both the photoacoustic signal and the ultrasonic signal may be collected and recorded without any problem despite the sequence being like this is that, in the case of the general optical-resolution photoacoustic mode, the relevant signal exists for a very short time only over a range of about 1 mm in depth from the surface of a subject and then quickly disappears. That is, this is possible because the photoacoustic signal in the optical-resolution mode is a much more transient signal than the ultrasonic signal that is co-registered with the photoacoustic signal. Therefore, the core concept underlying FIG. 3A and FIG. 3B is that an ultrasonic signal is collected as one concatenated signal immediately following a photoacoustic signal on one set of A-line signal rows obtained by giving only one trigger pulse to the digitizer 800 for each step signal of the scanning tip.

In a single-element ultrasonic transducer-based, mechanical scanning, integrated optical-resolution photoacoustic and ultrasonic endoscopes, probes, mini probes, or catheters, if a photoacoustic signal and an ultrasonic signal, which have significantly different signal levels before amplification (that is, just output from an ultrasonic transducer), are amplified by applying the same gain value to both signals based on the currently commercially available pulser/receiver (e.g., Olympus 5072PR) and then collected by using a single digitizer, that is, a data acquisition device, there could be a problem that one of the signals is saturated and lost beyond the preset dynamic range of the digitizer.

In addition, even in a process of acquiring each A-line signal, trigger pulses corresponding to the collection of photoacoustic and ultrasonic signals are applied alternately to the digitizer, and accordingly, a problem may occur in which a digitizer trigger frequency becomes twice the step frequency of the scanning tip of the relevant endoscope device, that is, the digitizer trigger frequency increases excessively.

For example, when the number of steps per rotation of the scanning tip of the endoscope device is assumed to be 800 and a rotational scan is performed 30 times per second, a step frequency becomes 24 kHz which is obtained by multiplying the number of steps by the rotational scan speed, and based on the existing circuit described above, the digitizer trigger frequency for alternately acquiring the photoacoustic and ultrasonic A-line signals eventually increases much further to twice, that is, 48 kHz. Of course, the increased digitizer trigger frequency is not always a problem, but when the step frequency is much higher and a data length of each A-line signal increases, the digitizer does not operate smoothly, leading to data loss.

The reason why two trigger pulses are transmitted to the digitizer at each step of the scanning tip is to distinguish the photoacoustic signal and the ultrasonic signal through each, and according to the method proposed by the present disclosure described above, the two signals may be sufficiently distinguished by using only one trigger pulse signal.

The reason why it is important to reduce the number of trigger pulses per time input to the digitizer is that, in general, each time a trigger pulse is input, the digitizer needs a certain amount of time to detect the trigger pulse and react thereto, and as a result, a continuously inputting electrical signal may not be collected during the reaction time.

Although an electric circuit for a photoacoustic-ultrasonic probe is mainly described above, the present disclosure is not limited thereto. For example, an electric circuit including components excluding a pulser and an ultrasonic transducer described above, and a photoacoustic-ultrasonic probe including the pulser and the ultrasonic transducer are also within the scope of the present disclosure.

As described above, a method for solving a problem of signal imbalance that may occur when collecting photoacoustic and ultrasonic signals simultaneously by using a single digitizer is presented, and a new trigger sequence form that may more effectively trigger a digitizer without data loss during a related execution process is presented. As far as the inventor knows, there is no example in the field of single transducer-based, mechanically scanning integrated optical-resolution photoacoustic and ultrasonic endoscopy technology which issues the problem of the present disclosure or presents a method for solving the problem.

As described above, the concept of separating two types of signals being transmitted through a single sensor by using a device, such as an RF switch, and amplifying & processing the separated signals with different gains may naturally be extended and applied when there are three or more types of signals to be processed or even when multiple types of signals being transmitted through multiple sensors have to be combined into a single port and collected and recorded.

### Industrial Applicability

An electric circuit for a photoacoustic-ultrasonic probe proposed by the present disclosure may be used for a photoacoustic-ultrasonic probe and utilized in a medical tomography endoscope device.

## Claims

1. An electric circuit for a photoacoustic-ultrasonic probe, the electric circuit comprising:
an ultrasonic T/R switch configured to receive a signal from an ultrasonic transducer;
an ultrasonic signal variable amplifier configured to amplify a received ultrasonic signal;
a photoacoustic signal variable amplifier configured to amplify a received photoacoustic signal; and
a first RF switch configured to receive a signal from the ultrasonic T/R switch and transmit the signal to the ultrasonic variable amplifier and the photoacoustic signal variable amplifier.

2. The electric circuit for the photoacoustic-ultrasonic probe of claim 1, further comprising a second RF switch configured to receive signals from the ultrasonic signal variable amplifier and the photoacoustic signal variable amplifier.

3. The electric circuit for the photoacoustic-ultrasonic probe of claim 1, wherein the first RF switch is configured to transmit an ultrasonic signal received from the ultrasonic T/R switch to the ultrasonic signal variable amplifier, and transmit a photoacoustic signal received from the ultrasonic T/R switch to the photoacoustic signal variable amplifier.

4. The electric circuit for the photoacoustic-ultrasonic probe of claim 1, wherein the ultrasonic T/R switch is configured to receive a signal while switching between a high impedance mode and a low impedance mode.

5. The electric circuit for the photoacoustic-ultrasonic probe of claim 4, wherein the ultrasonic T/R switch is configured to alternately operate in the high impedance mode and the low impedance mode.

6. The electric circuit for the photoacoustic-ultrasonic probe of claim 1, wherein a voltage level of an ultrasonic signal amplified by the ultrasonic signal variable amplifier corresponds to a voltage level of a photoacoustic signal amplified by the photoacoustic signal variable amplifier.

7. The electric circuit for the photoacoustic-ultrasonic probe of claim 1, further comprising a digitizer configured to receive and collect a signal from the second RF switch.

8. A photoacoustic-ultrasonic probe comprising:
the electric circuit for the photoacoustic-ultrasonic probe according to any one of claims 1 to 7;
a pulser configured to generate an electric pulse of a high voltage; and
an ultrasonic transducer configured to receive the electric pulse from the pulser, emit an ultrasonic wave, and detect a reflected wave.

9. The photoacoustic-ultrasonic probe of claim 8, wherein the ultrasonic transducer is further configured to detect a photoacoustic signal reflected from a subject toward which a laser pulse is emitted.

10. The photoacoustic-ultrasonic probe of claim 8, further comprising a low-noise preamplifier interposed between the ultrasonic transducer and the ultrasonic T/R switch.

11. The photoacoustic-ultrasonic probe of claim 8, further comprising a low-noise preamplifier interposed between the ultrasonic T/R switch and the first RF switch.
